Europäisches Patentamt

European Patent Office

Office européen des brevets

(11) Publication number: **0 482 540 A1**

# EUROPEAN PATENT APPLICATION

(21) Application number: **91117891.1**

(22) Date of filing: **21.10.91**

(51) Int. Cl.5: **A61K 47/48**

(30) Priority: **25.10.90 IT 2188090**

(43) Date of publication of application:
**29.04.92 Bulletin 92/18**

(84) Designated Contracting States:
**DE ES FR GB**

(71) Applicant: **LABORATORI BALDACCI Spa**
**Via San Michele degli Scalzi, 73**
**I-56100 Pisa(IT)**

(72) Inventor: **Fiume, Luigi**
**Via Dante, 7**
**I-40125 Bologna(IT)**
Inventor: **Chiricolo, Mariella**
**Via Ghirardini, 9**
**I-40100 Bologna(IT)**
Inventor: **Busi, Corrado**
**Via Berretta Rossa 68**
**I-40133 Bologna(IT)**
Inventor: **Mattioli, Alessandro**
**Via Ravenna, 14**
**I-40139 Bologna(IT)**

Inventor: **Spinelli Cosima**
**Via Jacopo Della Ouercia, 22**
**I-40128 Bologna(IT)**
Inventor: **Spinosa, Giulia**
**Via Frentana, 36**
**I-86039 Termoli (Campobasso)(IT)**
Inventor: **Bartolini, Giovanna**
**Via E. Malaguti, 27**
**I-40126 Bologna(IT)**
Inventor: **Minghetti, Luisa**
**Via Stradello, 22**
**I-48012 Bagnocavallo (Ravenna)(IT)**
Inventor: **Orlandi, Marina**
**Via Valdonica, 14**
**I-40126 Bologna(IT)**
Inventor: **Tomasi, Vittorio**
**Via Gerani, 20**
**I-44100 Ferrara(IT)**

(74) Representative: **Dragotti, Gianfranco et al**
**SAIC BREVETTI s.r.l. Viale Bianca Maria, 15**
**I-20122 Milano(IT)**

(54) **Novel derivatives of corticosteroids, process for their preparation and pharmaceutical composition containing them.**

(57) The conjugates of corticosteroids, like prednisolone and betamethasone, with human albumin show a more effective action at the level of the macrophages, by exploiting the activity characteristic of these corticosteroids without the characteristic side effects on other cellular systems.

For the preparation of these conjugates the corticosteroid, namely prednisolone or betamethasone, previously converted to the hemisuccinate is conjugated with human albumin by the method of the mixed anhydride, using isobutyl chlorocarbonate, with a molar ratio between corticosteroid and human albumin of between 15 and 19.

The resulting coniugate, preferably in a lyophilized form, is used for pharmaceutical compositions adapted for the parenteral administration, with dosages equal to or lower than the ususal ones for the starting corticosteroid of reference.

Fig.1

The present invention relates to novel derivatives of corticosteroids, particularly of those known with the names of prednisolone and betamethasone, to a process for their preparation and to the therapeutical compositions containing them as the active ingredient.

As it is well known the corticosteroids inhibit the production or the release of mediators produced from the macrophages, to which a key role is attributed in the inflammation mechanisms and the in immunitary response (prostaglandins, leucotrienes, etc.), (Schleimer R.P., ANN. Rev. Pharmacol.Toxicol., 25,381, (1985)).

Since the steroids are active also on other cellular systems involved in the phlogistic and immunitary processes, it is difficult to assess in what measure the activity of these drugs on the macrophages adds to their anti-inflammatory and immuno-suppressive effects. It is also known that the albumin, conjugated with small molecules is selectively absorbed from the cells of the macrophage system (Kruse H., Mc Master P.D., J, Exp. Med., 90,425, (1949)).

On the other side in vitro and in vivo experiments have shown that some toxins, such as amanitine and phalloidine, and some antiviral drugs, such as arabinoside cytosine and fluorodeoxyuridine, after conjugation with albumin have not lost the biological activity selectively applied in the macrophages. (Derenzini M. and Coll., Lab. Invest., 29 150, (1973); Barbanti-Brodano G., Fiume L., Nature New Biology 243, 281, (1973); Barbanti-Brodano G., Derenzini M. Fiume L., Nature, 248, 63, (1974); Balboni P.G. e coll., Nature 264, 181 (1976).

It has been now found that some corticosteroids, particularly prednisolone and betamethasone, if conjugated with human albumin, specifically enter into the macrophages thus suggesting that for some pathologies their therapeutical effects might be exploited withour the side effects characteristics of these drugs, thanks to the selectivity of cellular distribution after the administration.

In turn the process of the present invention for the preparation of the aforesaid conjugates comprises the carrying out of the conjugation between human albumin (HSA) and corticosteroid in form of hemisuccinate, as prepared in a per se known manner, according to the process of the mixed anhydride using isobutyl chlorocarbonate as described by Erlanger B.F. et al, J.Biol.Chem., 228, 713, (1957), and by determining by spectrophotometric way (Erlanger B.F., ibidem) on the resulting conjugate the molar ratio between corticosteroid and HSA, this ratio having been found in all the experimental preparations of between 15 and 19. This ratio remains unchanged also effecting the lyophilization of the conjugate and keeping it at room temperature for one year. The conjugate solution, after 24 hours at 37°C. does not show any release of free corticosteroid, thus demonstrating the stability of the bond between the corticosteroid and HSA; this bond is very probably a carboamido bond between the succinic group of the corticosteroid and the lysine amino groups of the protein.

For the pharmacological tests the conjugate preparations have been labelled with [125]I according to the method of Fraker and Speck (Fraker P.J., Speck Jr. J. C., Biochem. Biophys. Res. Commun. 80,849, (1978)).

The macrophages have been obtained from human monocytes isolated from peripheral blood (Orlandi M. and coll. Prostaglandins Leukot. Med., 18, 205, (1985)) and placed in culture for six days in RPMI 1640 (Flow) containing 10% human serum of the AB group. Fibrocytes of the cell line murine 3T3 have been cultivated in RPMI 1640 containing 10% of bovine fetal serum. For the experiments on the conjugate penetration, the cells (macrophages or fibroblasts) (4-6 x $10^6$) have been incubated in RPMI 1640 (without serum) in the presence of 300 ng/ml PRDN-([125]I)HSA (specific activity: 2.4 x $10^6$ cpm/ug). The cells have been collected at the time 0 and after one hour: after three washings with buffered saline solution of phosphates (PBS), they have been taken into solution with 2 ml of 6N NaOH and then the measurement has been effected of their radioactivity and of their protein content.

The concentrations of conjugate have been calculated on the values of intracellular radioactivity which have been obtained by subtracting the cpms calculated at the time 0 from those measured one our later (time 1h).The production of prostaglandin $E_2$ ($PGE_2$) and of thromboxane $A_2$ ($TXA_2$) has been induced in the macrophages by adding 10% of bovine fetal serum to the culture medium (Bartolini G. and coll., Biochim. Biophys. Acta, 876, 486, (1986)).

After 24 hours incubation the values of $PGE_2$ and of thromboxane $B_2$ (stabile metabolite of $TXA_2$) were assessed in the culture medium by RIA (Orlandi, ibidem). Table 1 shows the concentrations of PRDN-([125]I)-HSA in the macrophages and in the fibrocytes cultivated fro one hour in the presence of the conjugate. According to the literature sources stating that the chemically modified albumin molecules selectively enter in the macrophages, it was found that the concentration of conjugate in these cells is much higher than that in the fibrocytes.

## TABLE 1

### Concentration of PRDN-($^{125}$I)HSA in macrophages and cultivated fibroblasts.

| Cells | | PRDN-($^{125}$I)HSA (ng/ml cell protein) |
|---|---|---|
| Macrophages | 24,8 +/- 4,6[a] | |
| Fibroblasts | | 0.8 +/- 0.1 |

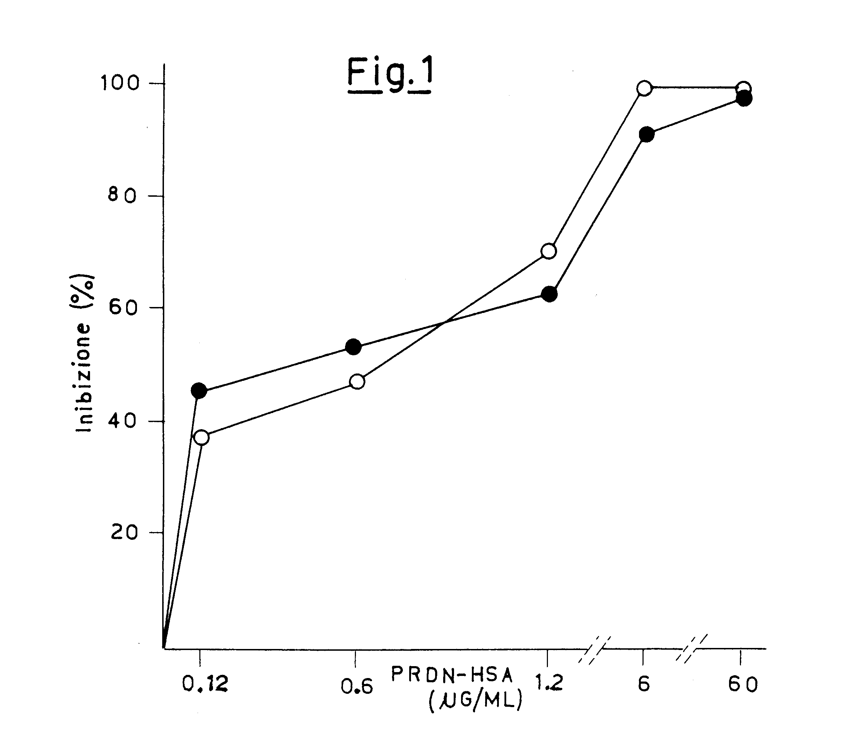

a: average value of the results (+/- standard error) of 4 tests. In the enclosed fig.1 it is shown that the conjugate PRDN-HSA inhibits the production of prostaglandin $E_2$ and of thromboxane $A_2$ induced in the in vitro cultivated macrophages by adding fresh bovine fetal serum.

The inhibition relates to the synthesis of prostaglandins $E_2$(*) and of thromboxane $A_2$(o) in the macrophages.Each point represents the average values of the results of four experiments. The standard errors of the percent inhibitions in the different experiments varied from +/- 0.6 to +/- 5.9 depending on the different concentrations of conjugate.For a conjugate concentration of 0.6 ug/ml, correponding to 0.05 ug/ml of coupled PRDN (prednisolone), a 50% inhibition is obtained of the production both of prostaglandins and of thromboxane.

Without imposing undue limits to the present invention it seems plausible to attribute the activity of the conjugate to an intracellular release of the drug from the carrier: this hypothesis is supported by the fact that, as it is well known, the albumin after the penetration into the macrophages enters the lysosomes and by that the steroids show their activity after having been bonded to a cytosolic receptor.It seems thus probable that the conjugate PRDN-HSA has the behaviour of a lysosometropic agent: the bond connecting the drug to the albumin is broken within the lysosomes and the free drug applies its pharmacological effect after crossing the lysosomial membrane.

This penetration selectivity of the conjugate into the macrophages has the consequence that the activity of the steroid takes thus place only at that level without the common side effects originating from the entering of the drug in other cellular systems.

Coming now to the betamethasone conjugate (BMT), it has been conjugated with HSA after its conversion in its hemisuccinate. This preparation is hereinafter described since the hemisuccinate, differently from that of prednisolone (PRDN), is not commercially available.

BMT (0.5 mmoles), succinic anhydride (4 mmoles) and 4-dimethylaminopyridine (0.17 mmoles) have been dissolved in 6 ml of anhydrous pyridine and maintained at room temperature. After 24 hours the thin layer chromatography on a silica gel plate (60F-254; Merck) with 2-propanol/concentrated ammonium hydroxide/water (60:30:10 v/v) has shown that practically all the BMT had been converted into the hemisuccinate thereof. At that point the pH of the reaction mixture has been lowered to the value 1 by means of HCl in order to precipitate the BMT hemisuccinate. After 1 hour at 0-4°C the mixture has been centrifugated , the supernatant being discarded. The BMT hemisuccinate has been dissolved in 12 ml water by adding $NaHCO_3$ powder and then precipitated again with HCl, collected and dried under vacuum (0,45 mmoles).

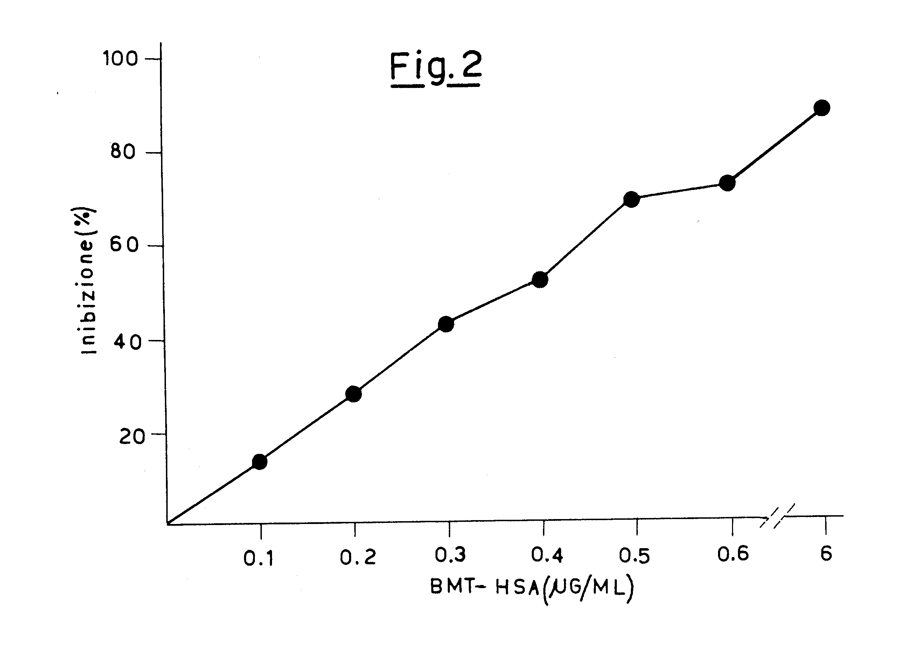

The BMT hemisuccinate has been conjugated to HSA using the same proceedings used for the preparation of the conjugate with PRDN hemisuccinate.The molar ratio drug/HSA, as spectrophometrically determined, was 15, 15 and 18 in three different preparations. The action of the BMT-HSA conjugate has been tested on the production of prostaglandins $E_2$ induced in macrophages activated in vitro by adding 10% fetal serum. The method used for the PRDN-HSA conjugate was used. The results reported in the fig.2, have shown that also the conjugate with BMT prevents $PGE_2$ from being formed. A 50% inhibition is attained with BMT-HSA concentrations of 0.3-0.4 ug/ml, corresponding to 0.02-0.03 ug of BMT /ml.

In experiments effected on the rat the in vivo penetration of the BMT-HSA conjugate in the parenchyma cells of the liver has been compared with that in the sinusoidal cells of the same organ, which belong to the system of the macrophage cells (Van Furth et al., Bull Wld Hlth Org. 46,854 (1972)).For these experiments a preparation of radioactive conjugate was used as obtained by binding BMT hemisuccinate to HSA labelled according to the method of Jentof and Darborn by using tritiated formaldehyde and following the method described in (Fiume et al. Cancer Drug Delivery 4, 11 (1987) (specific activity 1.2 x $10^6$ dpm/mg). The molar ratio drug/protein of the radioactive conjugate was 15. The experiments have been carried out exactly as described in Fiume et al., Cancer Drug Delivery 4, 11 (1987).

The results, reported in the table 2, show that the concentration of the conjugate in the sinusoidal cells is about hundred times higher than that of the parenchyma cells.

## TABLE 2

**Distribution of the BMT–Albumin conjugate in the parenchyma cells of the liver  30 minutes after its administration.**

| Compound | dose (ug/g) | parenchyma cells | sinusoidal cells |
|---|---|---|---|
| BMT-[$^3$H]HSA | 35 | 1.3 +/- 0.3 | 146.5 +/-1.2 |

The experiments have been carried out as described in Cancer Drug Delivery 4, 11 (1987).

The results have been calculated from the values of acid insoluble radioactivity of the cellular preparations. They have been expressed as a percentage of injected dpm x $10^3$ of cellular proteins. The data represent the average (+/- standard error) of the results of four experiments.

### Claims

1. Conjugates of a corticosteroid and human albumin having therapeutical activity.

2. Conjugates according to claim 1, characterized in that said corticosteroid is selected among prednisolone and betamethasone and the molar ratio between corticosteroid and human albumin is of between 15 and 19.

3. A process for the preparation of the conjugates according to the claims 1 and/or 2, characterized in that the corticosteroid, in form of hemisuccinate, is reacted with human albumin by the method of the mixed anhydride, using isobutyl chlorocarbonate.

4. Pharmaceutical compositions characterized by containing as the active ingredient a conjugate according to the claims 1 and/or 2.

Fig.1

EP 0 482 540 A1

Fig.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | PHARMACEUTICA ACTA HELVETIAE vol. 64, no. 12, 1989, ZURICH,SWITZERLAND pages 351 - 352; FIUME,L ET AL: 'A CONJUGATE OF PREDNISOLONE WITH ALBUMIN IS PHARMACOLOGICALLY ACTIVE IN MACROPHAGES' * the whole document * * | 1-4 | A 61 K 47/48 |
| Y | | 1-4 | |
| Y | EP-A-0 326 618   (NIPPON HYPOX LABORATORIES IN-CORPORATED) * page 5 * * | 1-4 | |
| D,Y | THE JOURNAL OF BIOLOGICAL CHEMISTRY vol. 228, no. 2, 1957, BALTIMORE,USA pages 713 - 727; ERLANG-ER,B,F: 'STEROID-PROTEIN CONJU-GATES:1.PREPARATION AND CHARACTERIZATION OF CONJUGATES OF BOVINE SERUM ALBUMIN WITH TES-TOSTERONE AND WITH CORTISONE' * the whole document * * | 1-4 | |
| Y | STEROIDS vol. 26, no. 5, 1975, SAN FRAN-SISCO,CALIFORNIA,USA pages 635 - 645; MIZUCHI,A ET AL: 'RADIOIMMUNOASSAY FOR PLASMA BE-TAMETHASONE 17-BENZOATE' * the whole document * * | 1-4 | TECHNICAL FIELDS SEARCHED (Int. Cl.5)  A 61 K |

The present search report has been drawn up for all claims

| Place of search | Date of completion of search | Examiner |
|---|---|---|
| The Hague | 14 January 92 | SITCH W.D.C. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document